# EUROPEAN PATENT APPLICATION

(11) **EP 4 454 673 A1**
(43) Date of publication of application: **30.10.2024**
(21) Application number: 24172315.4
(22) Date of filing: 25.04.2024
(51) Int. Cl.: A61M 1/02, A61M 1/34, A61M 1/36, A61M 1/38

(54) **BEDSIDE CELL PROCESSING SYSTEM HAVING A REINFUSION SYSTEM**

(30) Priority: 27.04.2023 US 202363498783 P
(71) Applicant: Fenwal, Inc., Lake Zurich, IL 60047 (US)
(72) Inventor: MIN, Kyungyoon, Lake Zurich, 60047 (US); WEGENER, Christopher J., Lake Zurich, 60047 (US)
(74) Representative: Maikowski & Ninnemann Patentanwälte Partnerschaft mbB

(57) **Abstract**

A bedside cell processing system including an apheresis system configured to collect from a patient a blood component comprising target cells, a cell processing system configured to receive the target cells from the apheresis system and to process the target cells, and a reinfusion system configured to receive the processed target cells and to return the processed cells to the patient. A method of operating a bedside cell processing system for a patient also is provided, including operating the apheresis system to collect from the patient via a collection flow path a blood component comprising target cells, operating a cell processing system to receive the target cells via a collected target cells flow path and to process the target cells, operating the reinfusion system to receive the processed target cells via a processed target cells flow path and to return the processed target cells to the patient.

## Description

### TECHNICAL FIELD

The present disclosure is generally directed to apparatus, systems and methods for processing or treating cells and returning the treated cells to a patient. More particularly, the present disclosure is directed to systems and methods that utilize an apheresis or cell collection system and cell processing system to process cells that are to be returned to the patient.

### BACKGROUND

Currently, a closed system for processing cells collected from blood or blood components of a patient and returning them to a patient is possible by utilizing an apheresis or cell collection system (hereinafter referred to as "apheresis system") and a cell processing system that are specially designed to work together. The patient remains connected to the specialized system for the entire time of the procedure. Accordingly, the patient typically is in a single location, commonly in an inclined chair or bed, which results in such systems being collectively referred to as a bedside cell processing system.

The apheresis system may separate and collect particular cells from blood, often referred to as blood components or target cells. The processing system then processes the target cells, and the processed cells are returned to the patient via the apheresis system. An example of this arrangement may be provided by use of the Amicus Extracorporeal Photopheresis (ECP) System (otherwise known as Amicus Blue), which is being developed and tested by Fenwal, Inc. of Lake Zurich, Illinois, which is an affiliate of Fresenius Kabi AG of Bad Homburg, Germany. The Amicus ECP System includes an Amicus apheresis system and a Phelix Photoactivation Device, together with specialized software that permits the Amicus apheresis system to be used to return the processed cells to the patient. Thus, the Phelix cell processing system can only be used to treat cells and return them to the patient if it is used with the Amicus apheresis system and special "return of the cells" software.

### SUMMARY

Apparatus, systems and methods are disclosed for collecting target cells from a patient, processing those cells and returning the processed/treated cells to the patient. However, by including an independent reinfusion system, the systems and methods may be agnostic to the capability of the apheresis system. Thus, by including an independent reinfusion system within the closed system that is connected to a patient, any apheresis system may be employed, as well as any suitable cell processing or treating system.

An apheresis system will collect the target cells. Apheresis or collection systems typically collect biological fluid such as blood or blood components by using a reusable processing apparatus ("hardware") and a disposable fluid circuit ("kit") adapted for mounting or other association with the reusable apparatus. After collection of the cells, the apheresis system will return to the patient any cells remaining in the disposable kit used with the apheresis system. This type of reinfusion of such remaining cells is typical of the reinfusion that may be routinely performed by an apheresis system relative to the collection process.

The collected target cells meanwhile are being processed via one or more cell processing unit(s). Examples of such units may include the Phelix Photoactivation Device, the Cue cell processing system, the Cue cell selection system or transduction system, a washing system, an incubation system, or other units suitable for processing cells for various purposes. The Phelix Photoactivation Device and Cue systems are sold by Fenwal, Inc. of Lake Zurich, Illinois, which is an affiliate of Fresenius Kabi AG of Bad Homburg, Germany. Somewhat similarly to the apheresis systems, cell processing systems may process cells by using a reusable processing apparatus ("hardware") and a disposable fluid circuit ("kit") adapted for mounting or other association with the reusable apparatus.

The time required to process cells depends on the procedure selected and the quantity of cells. As such, the processing of cells may take from a few minutes to many hours, during which the patient remains connected to the system. Connection of the patient to the system includes pre-connection of the patient to the separate reinfusion system. Accordingly, after cell processing, the processed cells will be reinfused into the patient via the infusion system. As with the apheresis and cell processing systems, the infusion system may use a reusable apparatus ("hardware") and a disposable fluid circuit ("kit") adapted for mounting or other association with the reusable apparatus.

The patient remains connected to the apheresis system and the reinfusion system during the procedure. At least three configurations for patient access by the apheresis system and reinfusion system are contemplated and disclosed herein. For example, in a first configuration, the apheresis system and reinfusion system may share a single patient access via a needle or catheter used with a single patient appendage and which is connected to the apheresis system to both collect biological fluid from the patient and to return to the patient cells remaining in the apheresis system kit, while also being connected to the reinfusion system to reinfuse into the patient processed cells. In a second example configuration, the apheresis system may have patient access via a first needle or catheter used with a first patient appendage and which is connected to the apheresis system to collect biological fluid. In the second example, the apheresis system and reinfusion system also may share patient access via a second needle or catheter used with a second patient appendage and which is connected to the apheresis system to return to the patient cells remaining in the apheresis system kit, while being connected to the reinfusion system to reinfuse into the patient processed cells. In a third example configuration, the apheresis system may have patient access via a first needle or catheter used with a first patient appendage and which is connected to the apheresis system to both collect biological fluid from the patient and to return to the patient cells remaining in the apheresis system kit, while the reinfusion system has separate patient access via a second needle or catheter used with a second patient appendage and which is connected to the reinfusion system to reinfuse into the patient processed cells.

Thus, patient access for the reinfusion system may be via pre-connection along the reinfusion line from the apheresis system, which also may be connected to or separate from the biological fluid collection line. Alternatively patient access for the reinfusion system may be via a reinfusion line that is separate from an apheresis system biological fluid collection and cell return reinfusion line.

Having a separate and independent reinfusion system provides an advantage in that the apheresis system selected need not be capable of receiving processed cells from the cell processing system and reinfusing such cells into the patient. Any apheresis system may be employed in the closed system to carry out the process. Also, the reinfusion system and the cell processing units of the cell processing systems may be connected in a closed system configuration, along with the apheresis system, via sterile connection devices.

The reinfusion system may include a microprocessor, memory, input device, output device, pump, one or more sensors and clamps, depending on the desired configuration. The sensors of the reinfusion system may be of types referred to as a venous pressure sensor, cell counter, hemolysis sensor, optical density sensor, or any other suitable sensor for monitoring the quality and/or quantity of cells, or other parameters of interest when reinfusing a patient with processed cells.

Thus, the use of a separate, independent reinfusion system facilitates flexibility and the potential use of different apheresis systems and different cell processing systems, while still permitting the convenience of a bedside cell processing system.

In a first aspect, the disclosure provides a bedside cell processing system, including an apheresis system configured to collect from a patient a blood component comprising target cells, a cell processing system configured to receive the target cells from the apheresis system and to process the target cells, and a reinfusion system configured to receive the processed target cells from the cell processing system and to return the processed cells to the patient.

In a second aspect, the disclosure provides a method of operating a bedside cell processing system for a patient, with the method comprising connecting an apheresis system to the patient via a collection flow path, connecting the apheresis system to a cell processing system via a collected target cells flow path, connecting the cell processing system to a reinfusion system via a processed target cells flow path, connecting the reinfusion system to the patient via a cell reinfusion system flow path, operating the apheresis system to collect from the patient via the collection flow path a blood component comprising target cells, operating the cell processing system to receive the target cells from the apheresis system via the collected target cells flow path and to process the target cells, operating the reinfusion system to receive the processed target cells from the cell processing system via the processed target cells flow path and to return the processed target cells to the patient via the reinfusion system flow path.

In light of the disclosure herein, it will be appreciated that bedside cell processing systems that include use of an independent reinfusion system, and method of use thereof, may be constructed and operated in advantageous, convenient and cost effective ways.

### BRIEF DESCRIPTION OF THE DRAWINGS

Figure 1 is a schematic diagram of a first example bedside cell processing system having a reinfusion system.
Figure 2 is schematic diagram of an example reinfusion system for use in the bedside cell processing system shown in Figure 1.
Figure 3 is a schematic diagram of a second example bedside cell processing system having a reinfusion system, such as is shown in Figure 2.
Figure 4 is a schematic diagram of a third example bedside cell processing system having a reinfusion system, such as is shown in Figure 2.

### DETAILED DESCRIPTION

The embodiments disclosed herein are for the purpose of providing an exemplary description of the present subject matter. They are, however, only exemplary, and the present subject matter may be embodied in various forms. Therefore, specific details disclosed herein are not to be interpreted as limiting the subject matter as defined in the accompanying claims.

It will be appreciated that a bedside cell processing system configured to include a separate reinfusion system for collection, processing and return of processed cells to a patient may be applicable for efficient and convenient use with a variety of apheresis systems and cell processing systems.

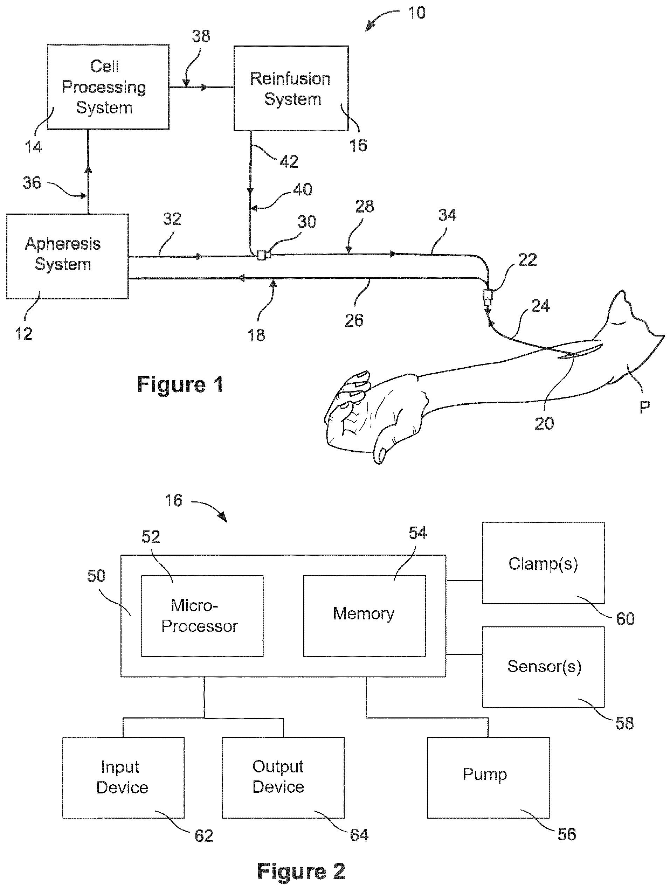

Figure 1 illustrates a first example embodiment of a bedside cell processing system 10. The bedside cell processing system 10 includes an apheresis system 12 configured to collect from a patient P a blood component comprising target cells, a cell processing system 14 configured to receive the target cells from the apheresis system 12 and to process the target cells, and a reinfusion system 16 configured to receive the processed target cells from the cell processing system 14 and to return the processed cells to the patient P. Thus, in use, the bedside cell processing system 10 is a closed system to which the patient remains connected throughout the procedure.

The apheresis system 12 may be any suitable type of apheresis or cell collection system. Some examples may be the Amicus, Alyx or Aurora systems sold by Fenwal, Inc. of Lake Zurich, Illinois, which is an affiliate of Fresenius Kabi AG of Bad Homburg, Germany. The apheresis system 12 further includes fluid communication access to the patient P via a collection flow path 18. The collection flow path 18 also includes a connection to the patient P via a needle or catheter 20. In the example embodiment shown, the collection flow path 18 further includes a Y-connector 22, and a first flow path 24 between the patient P and the Y-connector 22 and a second flow path 26 between the Y-connector 22 and the apheresis system 12. As previously noted, the apheresis or cell collection system 12 will collect biological fluid such as blood or blood components by using a reusable processing apparatus ("hardware") and a disposable fluid circuit ("kit") adapted for mounting or other association with the reusable apparatus. All connections to the kit of the apheresis system 12, such as the needle 20, are via sterile connectors, such as luer lock tubing connectors or suitable alternatives.

The example apheresis system 12 also includes fluid communication access to the patient P via an apheresis system reinfusion flow path 28 to return to the patient P cells that remain in the apheresis system 12 after at least a portion of the target cells are received by the cell processing system 14. The apheresis system reinfusion flow path may extend directly to the patient, or as shown in the first example embodiment, it may be connected to the collection line 18. Thus, in the example shown in Figure 1, the apheresis system reinfusion flow path 28 further includes a connection to the Y-connector 22 in the collection flow path 18, wherein the first flow path 24 also is used for reinfusion.

Indeed, the apheresis system reinfusion flow path 28 may include further connections, such as a Y-connector 30, and a third flow path 32 between the apheresis system 12 and the Y-connector 30 in the apheresis system reinfusion flow path 28 and a fourth flow path 34 between the Y-connector 30 in the apheresis system reinfusion flow path 28 and the Y-connector 22 in the apheresis system collection flow path 18.

The cell processing system 14 of the bedside cell processing system 10 includes fluid communication with the apheresis system 12 via a collected target cells flow path 36. The cell processing system 14 processes the target cells received from the apheresis system 12 using light treatment (photopheresis), cell separation, concentration and/or washing, or any other desired form of cell processing. The cell processing system 14 may be any suitable type of cell processing system, or any combination of cell processing system units. Some examples may be the Phelix Photoactivation Device, Cue or Lovo systems sold by Fenwal, Inc. of Lake Zurich, Illinois, which is an affiliate of Fresenius Kabi AG of Bad Homburg, Germany, or other suitable processing systems.

The cell processing system 14 will process the target cells by using a reusable processing apparatus ("hardware") and a disposable fluid circuit ("kit") adapted for mounting or other association with the reusable apparatus. All connections to the kit of the cell processing system 14, such as to fluid containers or to the apheresis system 12, are via sterile connectors, such as luer lock tubing connectors or suitable alternatives.

The reinfusion system 16 of the bedside cell processing system 10 further includes fluid communication with the cell processing system 14 via a processed target cells flow path 38. It will be appreciated that the reinfusion system 16 also includes fluid communication access to the patient P to return to the patient processed target cells via a reinfusion system flow path 40. The reinfusion system 16 will reinfuse cells by using a reusable processing apparatus ("hardware") and a disposable fluid circuit ("kit") adapted for mounting or other association with the reusable apparatus. All connections to the kit of the reinfusion system 16, such as to the cell processing system 14 or to the apheresis system reinfusion flow path or to a needle for connection to the patient, are via sterile connectors, such as luer lock tubing connectors or suitable alternatives

As noted with respect to the apheresis system reinfusion flow path 28 and shown in the first example embodiment in Figure 1, the reinfusion system flow path 40 may be connected to the apheresis system reinfusion flow path 28, utilizing a single patient access point for collection and reinfusion. Thus, as shown in the first example embodiment, the reinfusion system flow path 40 may include a fifth flow path 42 between the reinfusion system 16 and the Y-connector 30 in the apheresis system reinfusion flow path 28. In this example, it will be appreciated that the reinfusion system flow path 40 includes the fifth flow path 42 between the reinfusion system 16 and the Y-connector 30 in the apheresis system reinfusion flow path 28, the fourth flow path 34 between the Y-connector 30 in the apheresis system reinfusion flow path 28 and the Y-connector 22 in the apheresis system collection flow path 18 and the first flow path 24 between the Y-connector 22 in the apheresis system collection flow path 18 and the patient P. As previously noted, the connection to the patient P may be via a needle or catheter 20.

An example reinfusion system 16 is further illustrated in Figure 2 and includes a controller 50 having a microprocessor 52 and memory 54. The reinfusion system 16 also includes a pump 56. The reinfusion system 16 further preferably includes at least one sensor 58 and at least one clamp 60. An operator may operate the controller manually or engage an automated program mode via use of an input device 62 and view settings, performance or other data via an output device 64. The at least one sensor 58 may be configured in various forms to monitor desired parameters, such as a venous pressure sensor, a cell counter, a hemolysis sensor and/or an optical density sensor.

It will be appreciated that the bedside cell processing system 10 advantageously may be used in a method of operating a bedside cell processing system 10 for a patient P. The method includes connecting an apheresis system 12 to the patient P via a collection flow path 18, connecting the apheresis system 12 to a cell processing system 14 via a collected target cells flow path 36, connecting the cell processing system 14 to a reinfusion system 16 via a processed target cells flow path 38, connecting the reinfusion system 16 to the patient P via a reinfusion system flow path 40, operating the apheresis system 16 to collect from the patient P via the collection flow path 18 a blood component comprising target cells, operating the cell processing system 14 to receive the target cells from the apheresis system 12 via the collected target cells flow path 36 and to process the target cells, and operating the reinfusion system 16 to receive the processed target cells from the cell processing system 14 via the processed target cells flow path 38 and to return the processed target cells to the patient P via the reinfusion system flow path 40.

In utilizing the method, as described above, the collection flow path 18 may include a connection to the patient P via a needle or catheter 20. The method may further utilize an apheresis system 12 that includes an apheresis system reinfusion flow path 28 to return to the patient P cells that remain in the apheresis system 12 after at least a portion of the target cells are received by the cell processing system 14. The apheresis system reinfusion flow path 28 may include a portion of the apheresis system collection flow path 18. For example, the portion of the apheresis system collection flow path 18 included in the apheresis system reinfusion flow path 28 may include a connection to the patient P via a needle or catheter 20, such as via the Y-connector 22 and first flow path 24.

The reinfusion system flow path 40 may include a portion of the apheresis system reinfusion flow path 28 and a portion of the apheresis system collection flow path 18. For example, the portion of the apheresis system reinfusion flow path 28 included in the reinfusion system flow path 40 may include a Y-connector 30 and fourth flow path 34 in the apheresis system reinfusion flow path 28. Furthermore, the portion of the apheresis system collection flow path 18 that may be included in the reinfusion system flow path 40 may include the Y-connector 22, the first flow path 24 and the connection to the patient P via a needle or catheter 20.

It will be appreciated that operating the bedside cell processing system 10 includes processing the target cells received from the apheresis system 12 using light treatment (photopheresis), cell separation, concentration and/or washing, or other suitable forms of cell processing.

Operating the reinfusion system 16 includes having a controller 50 operate a pump 56 to deliver the processed target cells to the patient P. As described above, the controller 50 of the reinfusion system 16 may include a microprocessor 52 and memory 54. The reinfusion system 16 also may include at least one sensor 58 and at least one clamp 60. It will be appreciated that the at least one sensor 58 may include a venous pressure sensor, a cell counter, a hemolysis sensor and/or an optical density sensor, or other suitable sensors. Moreover, practicing the method of operating a bedside cell processing system 10 for a patient P may include the operator operating the reinfusion system 16 by entering parameters into the controller 50 via an input device 26, such as for manual or automated operation.

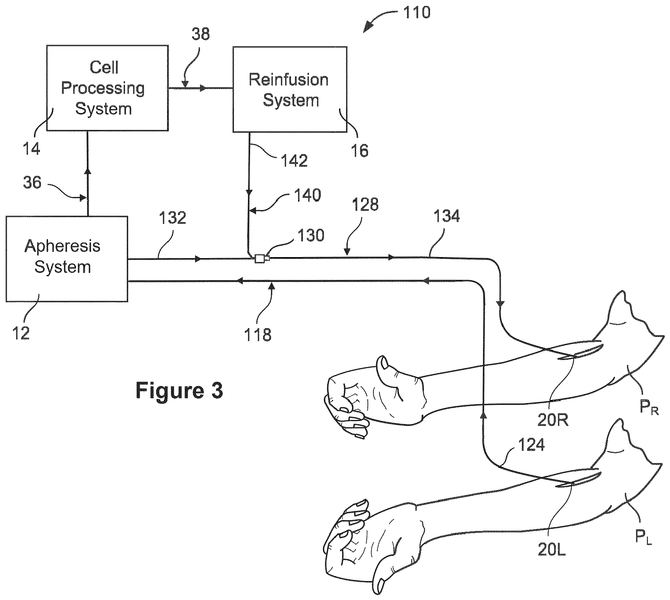

Figure 3 illustrates a second example embodiment of a bedside cell processing system 110. Similarly to the first example embodiment in Figure 1, the second example embodiment of a bedside cell processing system 110 includes an apheresis system 12 as previously described and configured to collect from a patient P a blood component comprising target cells, a cell processing system 14 as previously described and configured to receive the target cells from the apheresis system 12 and to process the target cells, and a reinfusion system 16 as previously described and configured to receive the processed target cells from the cell processing system 14 and to return the processed cells to the patient P. Thus, in use, the second example bedside cell processing system 110 also is a closed system to which the patient remains connected throughout the procedure.

In the second example bedside cell processing system 110, the apheresis system 12 further includes fluid communication access to the patient P via a collection flow path 118. The collection flow path 118 also includes a connection to the patient P_{L} (example left arm) via a needle or catheter 20L. In the second example embodiment shown, the collection flow path 118 further includes a first flow path 124 between the patient P_{L} and the apheresis system 12. As previously noted, the apheresis or cell collection system 12 will collect biological fluid such as blood or blood components by using a reusable processing apparatus ("hardware") and a disposable fluid circuit ("kit") adapted for mounting or other association with the reusable apparatus. All connections to the kit of the apheresis system 12, such as the needle 20L, are via sterile connectors, such as luer lock tubing connectors or suitable alternatives.

In the second example bedside cell processing system 110, the example apheresis system 12 also includes fluid communication access to the patient P via an apheresis system reinfusion flow path 128 to return to the patient P_{R} (example right arm) via a needle or catheter 20R cells that remain in the apheresis system 12 after at least a portion of the target cells are received by the cell processing system 14. The apheresis system reinfusion flow path may extend directly to the patient P_{R}, or as shown in the second example embodiment, it also may be connected to the reinfusion system 16. Thus, in the second example shown in Figure 2, the apheresis system reinfusion flow path 128 further includes a Y-connector 130, for the apheresis system reinfusion flow path 128 to also be used for reinfusion from the reinfusion system 16.

Indeed, the apheresis system reinfusion flow path 128 may include the Y-connector 130, and a second flow path 132 between the apheresis system 12 and the Y-connector 130 in the apheresis system reinfusion flow path 128 and a third flow path 134 between the Y-connector 130 in the apheresis system reinfusion flow path 128 and the needle or catheter 20R.

The cell processing system 14 of the bedside cell processing system 10 includes fluid communication with the apheresis system 12 via a collected target cells flow path 36. As previously noted, the cell processing system 14 processes the target cells received from the apheresis system 12 by using a reusable processing apparatus ("hardware") and a disposable fluid circuit ("kit") and may be any suitable type of cell processing system, or any combination of cell processing system units, with all connections to the kit of the cell processing system 14, such as to fluid containers or to the apheresis system 12, being via sterile connectors.

Similar to the first example, the reinfusion system 16 of the second example bedside cell processing system 110 further includes fluid communication with the cell processing system 14 via a processed target cells flow path 38. It will be appreciated that the reinfusion system 16 also includes fluid communication access to the patient P_{R} to return to the patient processed target cells via a reinfusion system flow path 140. As previously described, the reinfusion system 16 will reinfuse cells by using a reusable processing apparatus ("hardware") and a disposable fluid circuit ("kit"). All connections to the kit of the reinfusion system 16, such as to the cell processing system 14 or to the apheresis system reinfusion flow path or to a needle for connection to the patient, are via sterile connectors, such as luer lock tubing connectors or suitable alternatives.

As noted with respect to the apheresis system reinfusion flow path 28 shown in the first example embodiment in Figure 1, the reinfusion system flow path 40 may be connected to the apheresis system reinfusion flow path 28. However, in the second example embodiment in Figure 3, the reinfusion system flow path 140 is connected to the apheresis system flow path 128, and the two utilize a second, separate patient access point for all reinfusion. Thus, as shown in the second example embodiment in Figure 3, the reinfusion system flow path 140 may include a fourth flow path 142 between the reinfusion system 16 and the Y-connector 130 in the apheresis system reinfusion flow path 128. In this second example, it will be appreciated that the reinfusion system flow path 140 includes the fourth flow path 142 between the reinfusion system 16 and the Y-connector 130 in the apheresis system reinfusion flow path 128, the third flow path 134 between the Y-connector 130 in the apheresis system reinfusion flow path 128 and the patient P_{R}. As previously noted, the connection to the patient P_{R} may be via a needle or catheter 20R. The second example bedside cell processing system 110 may utilize the example reinfusion system 16, as illustrated in Figure 2, as above described.

Thus, it will be appreciated that the bedside cell processing system 110 advantageously may be used in a method of operating a bedside cell processing system 110 for a patient P. The method includes connecting an apheresis system 12 to the patient P_{L} via a collection flow path 118, connecting the apheresis system 12 to a cell processing system 14 via a collected target cells flow path 36, connecting the cell processing system 14 to a reinfusion system 16 via a processed target cells flow path 38, connecting the reinfusion system 16 to the patient P_{R} via a reinfusion system flow path 140, operating the apheresis system 16 to collect from the patient P_{L} via the collection flow path 118 a blood component comprising target cells, operating the cell processing system 14 to receive the target cells from the apheresis system 12 via the collected target cells flow path 36 and to process the target cells, and operating the reinfusion system 16 to receive the processed target cells from the cell processing system 14 via the processed target cells flow path 38 and to return the processed target cells to the patient P_{R} via the reinfusion system flow path 140.

In utilizing the method, as described above, the collection flow path 118 may include a connection to a first appendage of the patient P_{L} via a needle or catheter 20L. The method may further utilize an apheresis system 12 that includes an apheresis system reinfusion flow path 128 to return to a second appendage of the patient P_{R} cells that remain in the apheresis system 12 after at least a portion of the target cells are received by the cell processing system 14.

It will be appreciated that operating the second example bedside cell processing system 110 includes operating the reinfusion system 16, illustrated in Figure 2, in a manner similar to that described for the first example bedside cell processing system 10.

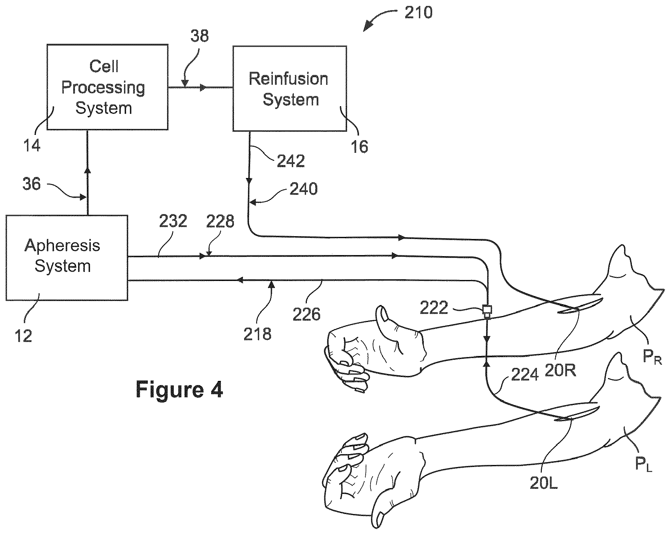

Figure 4 illustrates a third example embodiment of a bedside cell processing system 210. Similarly to the first example embodiment in Figure 1, the third example embodiment of a bedside cell processing system 210 includes an apheresis system 12 as previously described and configured to collect from a patient P a blood component comprising target cells, a cell processing system 14 as previously described and configured to receive the target cells from the apheresis system 12 and to process the target cells, and a reinfusion system 16 as previously described and configured to receive the processed target cells from the cell processing system 14 and to return the processed cells to the patient P. Thus, in use, the third example bedside cell processing system 210 also is a closed system to which the patient remains connected throughout the procedure.

In the third example bedside cell processing system 210, the apheresis system 12 further includes fluid communication access to the patient P via a collection flow path 218. The collection flow path 218 also includes a connection to the patient P_{L} (example left arm) via a needle or catheter 20L. In the third example embodiment shown, the collection flow path 218 further includes a Y-connector 222, and a first flow path 224 between the patient P_{L} and the Y-connector 222 and a second flow path 226 between the Y-connector 222 and the apheresis system 12. As previously noted, the apheresis or cell collection system 12 will collect biological fluid such as blood or blood components by using a reusable processing apparatus ("hardware") and a disposable fluid circuit ("kit") adapted for mounting or other association with the reusable apparatus. All connections to the kit of the apheresis system 12, such as the needle 20L, are via sterile connectors, such as luer lock tubing connectors or suitable alternatives.

The example apheresis system 12 also includes fluid communication access to the patient P_{L} via an apheresis system reinfusion flow path 228 to return to the patient P cells that remain in the apheresis system 12 after at least a portion of the target cells are received by the cell processing system 14. The apheresis system reinfusion flow path may extend directly to the patient, or as shown in the third example embodiment, it may be connected to the collection flow path 218. Thus, in the example shown in Figure 4, the apheresis system reinfusion flow path 228 further includes a connection to the Y-connector 222 in the collection flow path 218, wherein the first flow path 224 also is used for reinfusion from the apheresis system. Indeed, the apheresis system reinfusion flow path 228 may include a third flow path 232 between the apheresis system 12 and the Y-connector 222 in the apheresis system collection flow path 218.

The cell processing system 14 of the third example bedside cell processing system 210 includes fluid communication with the apheresis system 12 via a collected target cells flow path 36. As previously noted, the cell processing system 14 processes the target cells received from the apheresis system 12 by using a reusable processing apparatus ("hardware") and a disposable fluid circuit ("kit") and may be any suitable type of cell processing system, or any combination of cell processing system units, with all connections to the kit of the cell processing system 14, such as to fluid containers or to the apheresis system 12, being via sterile connectors.

Similar to the first example, the reinfusion system 16 of the third example bedside cell processing system 210 further includes fluid communication with the cell processing system 14 via a processed target cells flow path 38. However, it also will be appreciated that the reinfusion system 16 includes fluid communication access to the patient P_{R} (example right arm) via reinfusion system flow path 240, including a fourth flow path 242 and via a needle or catheter 20R to return to the patient processed target cells. As previously described, the reinfusion system 16 will reinfuse cells by using a reusable processing apparatus ("hardware") and a disposable fluid circuit ("kit"). All connections to the kit of the reinfusion system 16, such as to the cell processing system 14 or to the apheresis system reinfusion flow path or to a needle for connection to the patient, are via sterile connectors, such as luer lock tubing connectors or suitable alternatives.

Thus, it will be appreciated that the third example bedside cell processing system 210 advantageously may be used in a method of operating a bedside cell processing system 210 for a patient P. The method includes connecting an apheresis system 12 to the patient P_{L} via a collection flow path 218, connecting the apheresis system 12 to a cell processing system 14 via a collected target cells flow path 36, connecting the cell processing system 14 to a reinfusion system 16 via a processed target cells flow path 38, connecting the reinfusion system 16 to the patient P_{R} via a reinfusion system flow path 240, operating the apheresis system 16 to collect from the patient P_{L} via the collection flow path 218 a blood component comprising target cells, operating the cell processing system 14 to receive the target cells from the apheresis system 12 via the collected target cells flow path 36 and to process the target cells, and operating the reinfusion system 16 to receive the processed target cells from the cell processing system 14 via the processed target cells flow path 38 and to return the processed target cells to the patient P_{R} via the reinfusion system flow path 240.

In utilizing the method, as described above, the collection flow path 218 may include a connection to a first appendage of the patient P_{L} via a needle or catheter 20L. The method may further utilize an apheresis system 12 that includes an apheresis system reinfusion flow path 228 to return to the first appendage of the patient P_{L} cells that remain in the apheresis system 12 after at least a portion of the target cells are received by the cell processing system 14.

It will be appreciated that operating the third example bedside cell processing system 210 includes operating the reinfusion system 16, illustrated in Figure 2, to then return to the patient processed cells via the reinfusion system flow path 240.

Thus, an improved bedside cell processing system and methods of operating the system are disclosed herein. It will be appreciated that the reinfusion system of the disclosed bedside cell processing system may be a separate, independent system, which can be used with any alternative apheresis systems and any alternative cell processing systems. The description provided above, and the other aspects provided below, are intended for illustrative purposes, and are not intended to limit the scope of the disclosure to any particular apparatus, system or method described herein.

### Other Aspects

Aspect 1. A bedside cell processing system, comprising: an apheresis system configured to collect from a patient a blood component comprising target cells; a cell processing system configured to receive the target cells from the apheresis system and to process the target cells; and a reinfusion system configured to receive the processed target cells from the cell processing system and to return the processed cells to the patient.

Aspect 2. The bedside cell processing system of aspect 1, wherein the apheresis system further comprises fluid communication access to the patient via a collection flow path.

Aspect 3. The bedside cell processing system of aspect 2, wherein the collection flow path further comprises a connection to the patient via a needle or catheter.

Aspect 4. The bedside cell processing system of aspect 2, wherein the collection flow path further comprises a Y-connector, and a first flow path between the patient and the Y-connector and a second flow path between the Y-connector and the apheresis system.

Aspect 5. The bedside cell processing system of aspect 1, wherein the apheresis system further comprises fluid communication access to the patient to return to the patient cells that remain in the apheresis system after at least a portion of the target cells are received by the cell processing system via an apheresis system reinfusion flow path.

Aspect 6. The bedside cell processing system of aspect 4, wherein the apheresis system further comprises fluid communication access to the patient via an apheresis system reinfusion flow path to return to the patient cells that remain in the apheresis system after at least a portion of the target cells are received by the cell processing system.

Aspect 7. The bedside cell processing system of aspect 6, wherein the apheresis system reinfusion flow path further comprises a connection to the Y-connector in the collection flow path, wherein the first flow path also is used for reinfusion.

Aspect 8. The bedside cell processing system of aspect 7, wherein the apheresis or collection system reinfusion flow path further comprises a Y-connector, and a third flow path between the apheresis system and the Y-connector in the apheresis system reinfusion flow path and a fourth flow path between the Y-connector in the apheresis system reinfusion flow path and the Y-connector in the apheresis system collection flow path.

Aspect 9. The bedside cell processing system of aspect 1, wherein the cell processing system processes the target cells received from the apheresis system using light treatment, cell separation and/or washing.

Aspect 10. The bedside cell processing system of aspect 1, wherein the cell processing system further comprises fluid communication with the apheresis system via a collected target cells flow path.

Aspect 11. The bedside cell processing system of aspect 1, wherein the reinfusion system further comprises fluid communication with the cell processing system via a processed target cells flow path.

Aspect 12. The bedside cell processing system of aspect 1, wherein the reinfusion system further comprises fluid communication access to the patient to return to the patient processed target cells via a reinfusion system flow path.

Aspect 13. The bedside cell processing system of aspect 8, wherein the reinfusion system further comprises fluid communication access to the patient to return to the patient processed target cells via a reinfusion system flow path.

Aspect 14. The bedside cell processing system of aspect 13, wherein the reinfusion system flow path further comprises a fifth flow path between the reinfusion system and the Y-connector in the apheresis system reinfusion flow path.

Aspect 15. The bedside cell processing system of aspect 14, wherein the reinfusion system flow path further comprises the fifth flow path between the reinfusion system and the Y-connector in the apheresis system reinfusion flow path, the fourth flow path between the Y-connector in the apheresis system reinfusion flow path and the Y-connector in the apheresis system collection flow path and the first flow path between the Y-connector in the apheresis system collection flow path and the patient.

Aspect 16 The bedside cell processing system of aspect 1, wherein the reinfusion system further comprises a controller having a microprocessor and memory.

Aspect 17. The bedside cell processing system of aspect 16, wherein the reinfusion system further comprises a pump.

Aspect 18. The bedside cell processing system of aspect 17, wherein the reinfusion system further comprises at least one sensor and at least one clamp.

Aspect 19. The bedside cell processing system of aspect 18, wherein the reinfusion system further comprises an input device and an output device.

Aspect 20. The bedside cell processing system of aspect 19, wherein the at least one sensor further comprises a venous pressure sensor, a cell counter, a hemolysis sensor and/or an optical density sensor.

Aspect 21. A method of operating a bedside cell processing system for a patient, the method comprising: connecting an apheresis system to the patient via a collection flow path; connecting the apheresis system to a cell processing system via a collected target cells flow path; connecting the cell processing system to a reinfusion system via a processed target cells flow path; connecting the reinfusion system to the patient via a reinfusion system flow path; operating the apheresis system to collect from the patient via the collection flow path a blood component comprising target cells; operating the cell processing system to receive the target cells from the apheresis system via the collected target cells flow path and to process the target cells; and operating the reinfusion system to receive the processed target cells from the cell processing system via the processed target cells flow path and to return the processed target cells to the patient via the reinfusion system flow path.

Aspect 22. A method of aspect 21, wherein the collection flow path further comprises a connection to the patient via a needle or catheter.

Aspect 23. A method of aspect 21, wherein the apheresis system further comprises an apheresis system reinfusion flow path to return to the patient cells that remain in the apheresis system after at least a portion of the target cells are received by the cell processing system.

Aspect 24. A method of aspect 23, wherein the apheresis system reinfusion flow path includes a portion of the apheresis system collection flow path.

Aspect 25. A method of aspect 24, wherein the portion of the apheresis system collection flow path included in the apheresis system reinfusion flow path further comprises a connection to the patient via a needle or catheter.

Aspect 26. A method of aspect 23, wherein the reinfusion system flow path includes a portion of the apheresis system reinfusion flow path and a portion of the apheresis system collection flow path.

Aspect 27. A method of aspect 26, wherein the portion of the apheresis system collection flow path included in the apheresis system reinfusion flow path further comprises a connection to the patient via a needle or catheter.

Aspect 28. A method of aspect 21, wherein operating the bedside cell processing system further comprises processing the target cells received from the apheresis system using light treatment, cell separation, concentration and/or washing.

Aspect 29. A method of aspect 21, wherein operating the reinfusion system further comprises having a controller operate a pump to deliver the processed target cells to the patient.

Aspect 30. A method of aspect 29, wherein controller of the reinfusion system further comprises a microprocessor and memory.

Aspect 31. A method of aspect 30, wherein the reinfusion system further comprises at least one sensor and at least one clamp.

Aspect 32. A method of aspect 31, wherein the at least one sensor further comprises a venous pressure sensor, a cell counter, a hemolysis sensor and/or an optical density sensor.

Aspect 33. A method of aspect 31, wherein operating the reinfusion system further comprises entering parameters into the controller via an input device.

## Claims

1. A bedside cell processing system, comprising:
an apheresis system configured to collect from a patient a blood component comprising target cells;
a cell processing system configured to receive the target cells from the apheresis system and to process the target cells; and
a reinfusion system configured to receive the processed target cells from the cell processing system and to return the processed cells to the patient.

2. The bedside cell processing system of claim 1, wherein the apheresis system further comprises fluid communication access to the patient via a collection flow path.

3. The bedside cell processing system of claim 2, wherein the collection flow path further comprises a connection to the patient via a needle or catheter.

4. The bedside cell processing system of claim 2, wherein the collection flow path further comprises a Y-connector, and a first flow path between the patient and the Y-connector and a second flow path between the Y-connector and the apheresis system.

5. The bedside cell processing system of claim 1, wherein the apheresis system further comprises fluid communication access to the patient to return to the patient cells that remain in the apheresis system after at least a portion of the target cells are received by the cell processing system via an apheresis system reinfusion flow path.

6. The bedside cell processing system of claim 4, wherein the apheresis system further comprises fluid communication access to the patient via an apheresis system reinfusion flow path to return to the patient cells that remain in the apheresis system after at least a portion of the target cells are received by the cell processing system.

7. The bedside cell processing system of claim 6, wherein the apheresis system reinfusion flow path further comprises a connection to the Y-connector in the collection flow path, wherein the first flow path also is used for reinfusion.

8. The bedside cell processing system of claim 7, wherein the apheresis or collection system reinfusion flow path further comprises a Y-connector, and a third flow path between the apheresis system and the Y-connector in the apheresis system reinfusion flow path and a fourth flow path between the Y-connector in the apheresis system reinfusion flow path and the Y-connector in the apheresis system collection flow path.

9. The bedside cell processing system of claim 1, wherein the cell processing system processes the target cells received from the apheresis system using light treatment, cell separation and/or washing.

10. The bedside cell processing system of claim 1, wherein the cell processing system further comprises fluid communication with the apheresis system via a collected target cells flow path.

11. The bedside cell processing system of claim 1, wherein the reinfusion system further comprises fluid communication with the cell processing system via a processed target cells flow path.

12. The bedside cell processing system of claim 1, wherein the reinfusion system further comprises fluid communication access to the patient to return to the patient processed target cells via a reinfusion system flow path.

13. The bedside cell processing system of claim 1, wherein the reinfusion system further comprises a controller having a microprocessor and memory.

14. The bedside cell processing system of claim 13, wherein the reinfusion system further comprises a pump.

15. The bedside cell processing system of claim 14, wherein the reinfusion system further comprises at least one sensor and at least one clamp.

16. The bedside cell processing system of claim 15, wherein the reinfusion system further comprises an input device and an output device.

17. The bedside cell processing system of claim 16, wherein the at least one sensor further comprises a venous pressure sensor, a cell counter, a hemolysis sensor and/or an optical density sensor.
